# NEUE EUROPÄISCHE PATENTSCHRIFT

(11) **EP 1 648 485 B2**
(45) Veröffentlichungstag und Bekanntmachung des Hinweises auf die Entscheidung über den Einspruch: **24.09.2014**
(45) Hinweis auf die Patenterteilung: 20.04.2011
(21) Anmeldenummer: 04762485.3
(22) Anmeldetag: 22.07.2004
(51) Int. Cl.: A61K 38/37, A61K 9/19

(54) **FORMULIERUNG FÜR PROTEINARZNEIMITTEL OHNE ZUSATZ VON HUMANEM SERUMALBUMIN (HSA)**
FORMULATION FOR A PROTEIN PHARMACEUTICAL WITHOUT ADDED HUMAN SERUM ALBUMIN (HSA)
FORMULATION DESTINEE A UN AGENT PHARMACEUTIQUE PROTEIQUE NE CONTENANT PAS D'ALBUMINE SERIQUE HUMAINE AJOUTEE (HSA)

(30) Priorität: 22.07.2003 DE 10333317
(43) Veröffentlichungstag der Anmeldung: 26.04.2006
(73) Patentinhaber: Merz Pharma GmbH & Co. KGaA, 60318 Frankfurt am Main (DE)
(72) Erfinder: FREVERT, Jürgen, 10625 Berlin (DE)
(74) Vertreter: Dehmel, Albrecht
(86) Internationale Anmeldenummer: PCT/DE2004/001635
(87) Internationale Veröffentlichungsnummer: WO 2005/007185

(56) Entgegenhaltungen:
- US-A- 5 512 547
- US-A- 5 565 427
- H. DOOTZ: "Rote Liste 2003" 1. Januar 2003 (2003-01-01), ROTE LISTE SERVICE GMBH , AULENDORF , XP002319956 Absätze [16020], [16031]

## Beschreibung

Die vorliegende Erfindung betrifft eine Zusammensetzung aus niedermolekularen, nicht-peptidischen Substanzen, die Protein-Wirkstoffe, in Arzneimitteln formuliert, stabilisiert und bei der dabei auf die Verwendung von HSA verzichtet wird. Die vorliegende Erfindung betrifft weiterhin eine pharmazeutische Zusammensetzung, die neben diesem Protein-Wirkstoff auch die Zusammensetzung aus niedermolekularen, nicht-peptidischen Substanzen enthält.

Die Entwicklung der Gentechnik liefert eine Vielzahl von neuartigen Arzneimitteln, deren Wirkstoffe Proteine darstellen. Im Vergleich zu herkömmlichen Arzneimitteln, deren Wirkstoffe aus niedermolekularen Substanzen bestehen, zeichnen sich die hochmolekularen Proteine durch eine hohe Wirksamkeit bei geringer Substanzmenge aus und werden deshalb in sehr geringen Konzentrationen bzw. Dosierungen eingesetzt.

Bei solchen geringen Konzentrationen bzw. Dosierungen sehen sich die Hersteller von Arzneimitteln mit einem Problem konfrontiert. Proteine haben nämlich die Eigenschaft, sich an feste Oberflächen zu heften. Aufgrund dieser Adsorption kann ein großer Teil der Menge an eingesetztem Protein-Wirkstoff verloren gehen. Dabei ist dieser Effekt natürlich um so schwerwiegender, je geringer die Konzentration des zu applizierenden Protein-Wirkstoffes ist. Ohne eine geeignete Formulierung kann der Protein-Wirkstoff sogar vollständig verloren gehen.

Ein weiteres Problem dieser Arzneimittel bzw. der Protein-Wirkstoffe besteht in der hohen Instabilität von Proteinen. Sie können z.B. leicht oxidiert (Cystein-, Methionin-Reste) bzw. desaminiert werden (Asparagin), oder sie werden in Fragmente gespalten bzw. aggregieren zu höheren Komplexen. Eine effiziente Formulierung sollte derartige Verluste an Protein-Wirkstoff vermeiden und ein stabiles Produkt gewährleisten.

Da die Bindung von Proteinen an Oberflächen unspezifisch ist, lässt sich der Verlust an Wirkstoff durch Zugabe eines anderen (unspezifischen) Proteins in hohem Überschuß verhindern. Da das zusätzliche Protein möglichst keinerlei pharmakologische Wirkung haben und auch die Produktion von Antikörpern nicht stimulieren sollte, wird für diese Zwecke zur Zeit humanes Serumalbumin (HSA) verwendet, das außerdem preisgünstig zu beziehen ist, da es in großen Mengen als Plasmaersatz eingesetzt wird. So sind derzeit eine Reihe von Arzneimitteln (verschiedene Interferone, Wachstumsfaktoren, Gerinnungsfaktoren, Botulinum-Toxine und Vakzine) auf dem Markt, die als Stabilisator HSA enthalten.

Bei HSA handelt es sich um ein aus menschlichem Blut gewonnenes Produkt, das somit trotz vorgeschriebener Prüfung (z.B. durch Viren) kontaminiert sein und für eine Übertragung einer Erkrankung auf den Empfänger des HSA-haltigen Arzneimittels sorgen kann (zumal von Zeit zu Zeit neue Pathogene auftauchen (können), die durch Tests nicht rechtzeitig erfaßt werden können). Die Zulassungsbehörden drängen deshalb darauf, HSA bei Neuzulassungen von Arzneimitteln zu ersetzen. Aus diesem Grunde sollte HSA in Formulierungen von Arzneimitteln nicht verwendet werden - sofern es durch andere Substanzen ersetzt werden kann.

Humanes Serumalbumin (HSA) eignet sich aus verschiedenen Gründen besonders für die Formulierung eines Protein-Wirkstoffes. Es ist ein Protein und kann somit alle unspezifischen Reaktionen am Protein-Wirkstoff hemmen bzw. neutralisieren. Das gilt insbesondere für Reaktionen an Grenzflächen (Flüssig-Fest, Flüssig-Gas), die zu einer Denaturierung des Wirkstoffes führen können (Henson et al (1970), Colloid Interface Sci 32, 162-165). Die Anwesenheit von HSA schützt somit vor Denaturierung. Proteine haben außerdem eine Affinität zu Oberflächen, an die sie mittels hydrophober Wechselwirkungen unspezifisch binden (Norde W. (1995) Cells Mater 5, 97-112). Mit einem Überschuß an HSA lassen sich diese Bindungsplätze an Oberflächen absättigen, so dass der Protein-Wirkstoff in Lösung bleibt, was insbesondere zwingend erforderlich ist, wenn die Dosis des Protein-Wirkstoffes gering ist.

Des weiteren schützt die Anwesenheit von HSA vor Denaturierungsprozessen während der Abfüllung und gegebenenfalls Lyophilisierung sowie bei der Lagerung des Arzneimittels (z.B. vor oxidativen Abbauprozessen oder vor einer Desaminierung von Asparagin).

Ein so den Wirkstoff schützendes Protein sollte natürlich selbst keine pharmakologische Wirkung aufweisen, eine Bedingung, die von HSA erfüllt wird. Da es ein menschliches Protein ist, sollte HSA auch nicht als Antigen wirken, d.h. es sollte die Antikörper-Produktion nicht stimulieren. Da HSA aber aus Blut isoliert und mittels physikalisch-chemischer Verfahren gereinigt wird, kann man nicht grundsätzlich ausschließen, dass durch den Reinigungsprozeß Neoepitope, also neue antigene Strukturen, entstehen, gegen die der Empfänger des Gemisches aus HSA und Protein-Wirkstoff Antikörper bildet. In diesem Fall könnte es zu unerwünschten Nebenreaktionen kommen. Wegen möglicher Nebenreaktionen ist die Verwendung eines anderen Proteins bzw. eines Gemisches von Oligopeptiden nicht wünschenswert.

Grundsätzlich kommt auch Gelatine als Stabilisator in Frage. Dabei handelt es sich aber um ein tierisches Protein, das immunologische Reaktionen hervorrufen und auch Träger pathogener Agentien sein könnte.

Die Verwendung von HSA bzw. einem anderen geeigneten Stabilisator für den Protein-Wirkstoff ist also besonders wichtig bei Arzneimitteln, deren Protein-Wirkstoffe in äußerst geringen Dosen enthalten sind bzw. verabreicht werden. Denn Proteine sind vor allem in geringen Konzentrationen äußerst labil und binden außerdem sofort an verfügbare unspezifische Bindungsplätze. Als Folge davon sind sie für die therapeutische Verwendung verloren. Als Beispiele für einen Protein-Wirkstoff, der in äußerst geringen Dosen eingesetzt wird, seien die Neurotoxine von *Clostridium botulinum* genannt. Diese hochwirksamen Proteine wirken in geringsten Mengen (sie sind bzw. das Neurotoxin von *Clostridium botulinum* Typ A ist von allen bisher entwickelten Arzneimitteln dasjenige, das mit der geringsten Dosierung (500 pg/Glasfläschchen) verabreicht wird). Dieses äußerst geringe Quantum an Protein geht verloren, sofern kein schützendes Agens verwendet wird.

US 5,565,427 offenbart eine Lösung mit Faktor VIII: C-Aktivität, die eine Aminosäure oder eines seiner Salze oder Derivate enthält, und im geeigneten Fällen ein Detergens oder ein organisches Polymer.

Aus der Schilderung des Standes der Technik, der im wesentlichen als ein solches schützendes Agens nur HSA beschreibt, geht hervor, daß ein Bedarf besteht, Alternativen zu HSA als Stabilisator für Protein-Wirkstoffe in Arzneimitteln bereitzustellen. Dementsprechend haben sich die Erfinder die Aufgabe gestellt, eine Zusammensetzung zu entwickeln, die Protein-Wirkstoffe in Arzneimitteln wenigstens so gut schützt/stabilisiert, wie dies HSA tut.

Die gestellte Aufgabe hat der Erfinder dadurch gelöst, dass er eine Zusammensetzung aus niedermolekularen, nicht-peptidischen Substanzen entwickelte, die Protein-Wirkstoffe, formuliert in Arzneimitteln, stabilisiert und bei der dabei auf die Verwendung von HSA verzichtet wird. Diese Zusammensetzung wird nachfolgend auch als "Zusammensetzung zur Stabilisierung" bezeichnet. Die Erfindung betrifft eine pharmazeutische Zusammensetzung nach Anspruch 1.

Die vorliegende Erfindung offenbart eine Zusammensetzung, frei von Fremdprotein, die mit Protein-Wirkstoffen zu Arzneimitteln formuliert werden kann. Diese Zusammensetzung zur Stabilisierung basiert vorzugsweise auf niedermolekularen Substanzen, die gemäß der Europäischen Pharmakopoe (Ph.Eur.) hergestellt werden und als pharmazeutische Hilfsstoffe zugelassen sind. Sie erlaubt es insbesondere, auf HSA zu verzichten und gewährleistet nicht nur eine stabile Lagerung der Protein-Wirkstoffe bzw. des Arzneimittels ohne Wirkstoffverlust, sondern beseitigt auch das Risiko, dass das entsprechend verabreichte Arzneimittel mit infektiösen Agentien kontaminiert ist. Überraschend ist, dass solche niedermolekularen und "einfachen" Substanzen, wie sie erfindungsgemäß für die Zusammensetzung zur Stabilisierung verwendet werden, die gewünschte Leistungsfähigkeit aufweisen und HSA ersetzen können.

Die erfmdungsgemäße Zusammensetzung zur Stabilisierung ersetzt das HSA durch eine Kombination verschiedener niedermolekularer nebenwirkungsfreier Substanzen, die vor dem Verlust von Protein-Wirkstoff durch Adsorption an Oberflächen sowie durch Denaturierung und chemische Abbauprozesse der gelösten oder lyophilisierten Protein-Wirkstoffe schützen. Darüber hinaus verhindert die Zusammensetzung zur Stabilisierung den Abbau des Wirkstoffes bei der Lagerung über einen Zeitraum > 6 Monaten auch bei erhöhter Temperatur.

Die erfindungsgemäße Zusammensetzung zur Stabilisierung weist die in Anspruch 1 angegebenen Komponenten auf, das sind:
a) eine oberflächenaktive Substanz, vorzugsweise ein nicht-ionisches Detergens (Tensid),
   und
b) ein Gemisch von mindestens drei Aminosäuren, wobei die mindestens das Gemisch aus (i) Asp, Asn u. Glu; (ii) Asp, Asn u. Gln; (iii) Asp, Glu u. Gln; (iv) Asn, Glu u. Gln; oder (v) Asp, Asn, Glu u. Gln besteht.

Die erfindungsgemäße Zusammensetzung zur Stabilisierung enthält gemäß einer weiteren bevorzugten Ausführungsform eine oder mehrere der folgenden weiteren Komponenten:
c) ein Disaccharid, vorzugsweise Sucrose (Saccharose, Rohrzucker), Trehalose oder Lactose;
d) Ethylendiamintetraessigsäure (EDTA), vorzugsweise in Form eines ihrer Salze wie des Na₄-EDTA.

Bevorzugte erfindungsgemäße Zusammensetzungen zur Stabilisierung enthalten entweder die Komponenten a), b) und c), oder die Komponenten a), b) und d), oder die Komponenten a), b) c) und d). Alle diese bevorzugten Zusammensetzungen sind entweder in wässrigen Medien löslich oder sie sind wässrige Lösungen.

Es ist vorteilhaft, dass alle in der Zusammensetzung zur Stabilisierung verwendeten Substanzen als Hilfsstoffe für pharmazeutische Zubereitungen zugelassen und somit auch toxikologisch eingehend untersucht worden sind, das heißt, dass sie ohne weitere Prüfungen in die erfindungsgemäße Zusammensetzung zur Stabilisierung eingemischt werden können. Überraschend war, dass eine genau definierte Zusammenstellung dieser einfachen Substanzen die gewünschte Leistungsfähigkeit aufweist, nämlich eine stabile Serumalbumin-freie Formulierung für Protein-Wirkstoffe zu liefern.

Der Wirkstoff bzw. Protein-Wirkstoff wird vorzugsweise in einer wässrigen Lösung zubereitet (gelöst), die die Komponenten a) und b) und ggf. auch c) und/oder d) enthält. Sie kann anschließend lyophilisiert werden. Wenn sie tatsächlich lyophilisiert werden soll, ist die vorherige Zugabe von Komponente c) besonders vorteilhaft. Nach dem Lyophilisieren liegt die pharmazeutische Zusammensetzung als Pulver vor, das (vorzugsweise mit Wasser für Injektionszwecke (WFI)) rekonstituiert werden kann.

Dementsprechend liegt die pharmazeutische Zusammensetzung vorzugsweise als gefrier- oder Vakuum-getrocknetes Pulver vor, das in wässrigen Medien löslich ist. Vor dem therapeutischen Einsatz wird das Lyophilisat bzw. Pulver vorzugsweise mit Wasser für Injektionszwecke (WFI) rekonstituiert. Die pharmazeutische Zusammensetzung kann aber auch in flüssiger Form, vorzugsweise als wässrige Lösung, vorliegen.

Die erfindungsgemäße pharmazeutische Zusammensetzung der vorliegenden Erfindung enthält neben den oben genannten Komponenten a) und b) als Protein einen Gerinnungsfaktor wie den Faktor VIII (das antihämophile Globulin) ein Enzym wie eine Urokinase oder Streptokinase, einen Plasminogenaktivator oder ein hochreines Neurotoxin (zur Definition von "hochreines Neurotoxin" siehe weiter unten) bzw. einen Neurotoxin-Komplex aus *Clostridium botulinum,* insbesondere aus *Clostridium botulinum* der Typen A, B, C, D, E, F oder G ist. Da die clostridiellen Toxine, insbesondere in ihrer hochreinen Form, in geringsten Mengen als Arzneimittel formuliert werden, sind sie die bevorzugten Protein. Besonders bevorzugt sind die hochreinen Neurotoxine der Typen A und B.

Weitere bevorzugte pharmazeutische Zusammensetzungen der vorliegenden Erfindung enthalten neben den oben genannten Komponenten a) und b) und dem Wirkstoff zusätzlich die oben definierte Komponente c), die oben definierte Komponente d), oder diese beiden Komponenten gemeinsam.

Bei den erfindungsgemäßen Zusammensetzungen sind wenigstens drei (Asparaginsäure, Asparagin, Glutaminsäure; Asparaginsäure, Asparagin, Glutamin; Asparaginsäure, Glutaminsäure, Glutamin; Asparagin, Glutaminsäure, Glutamin) dieser vier Aminosäuren, oder sogar alle vier (Asparaginsäure, Asparagin, Glutaminsäure und Glutamin) enthalten. Vorzugsweise werden die einzelnen Aminosäuren in einer Konzentration von 20 bis 200 mM, besser 20 bis 100 mM, insbesondere 50 mM, verwendet. Das entspricht bei einer Abfüllung von 0.5 ml Ausgangs-Lösung nach Trocknung einer Menge von 1.3 mg bis 14.7 bzw. 1.3 bis 7.4 mg, insbesondere etwa 3.7 mg pro Aminosäure im Pulver.

Bei einer weiteren bevorzugten Ausführungsform der Zusammensetzungen der vorliegenden Erfindung ist die oberflächenaktive Substanz (das Tensid) ein nicht-ionisches Detergens, vorzugsweise ein Polysorbat (wie Polysorbat 20 oder Polysorbat 80) oder ein Poloxamer (wie Poloxamer 184 oder Poloxamer 188). Wenn die pharmazeutische Zusammensetzung in flüssiger Form vorliegt, beträgt der Anteil des Polysorbats vorzugsweise 0.01 bis 0.5 Gew.-%, bevorzugt ist ein Anteil von 0.2 Gew.-%. Das entspricht nach z.B. einer Gefriertrocknung von 0.5 ml Ausgangs-Lösung einer Menge von 0.05 bis 2.5 mg, bevorzugt 1 mg Polysorbat.

Bei einer noch weiteren bevorzugten Ausführungsform der Zusammensetzungen der vorliegenden Erfindung ist die das Disaccharid Saccharose, Trehalose oder Lactose. Saccharose ist besonders bevorzugt. Liegt die erfindungsgemäße pharmazeutische Zusammensetzung als Lösung vor, enthält sie vorzugsweise 2 bis 10 Gew.-%, bevorzugterweise 5 Gew.-% des Disaccharids, speziell der Saccharose.

Weiterhin bevorzugt ist, wie schon beschrieben, die Verwendung von Komponente d), einem Komplexbildner. Dieser übt einen zusätzlichen stabilisierenden Effekt aus. Die Konzentration der Ethylendiamintetraessigsäure in der Ausgangs-Lösung der pharmazeutischen Zusammensetzung beträgt vorzugsweise 0.1 bis 1.0 mM, insbesondere 5 mM.

Beide Typen von Zusammensetzungen gemäß vorliegender Erfindung weisen bevorzugterweise einen pH-Wert von 5.0 bis 8.5, besonders bevorzugterweise einen pH-Wert von 6.0 bis 8.0, insbesondere von 6.0 bis 7.0 bzw. 6.5, auf. Der pH-Wert wird gegebenenfalls, sofern nötig bzw. erwünscht, mit NaOH eingestellt.

Die eingangs bereits erwähnten Neurotoxine von *Clostridium botulinum,* insbesondere die Neurotoxine von *Clostridium botulinum* der Typen A und B, muß für pharmazeutische Zwecke in sehr geringen Dosierungen formuliert werden. Damit läßt sich die Qualität der erfindungsgemäßen Zusammensetzung zur Stabilisierung besonders gut an diesem extrem schwierig zu handhabenden Wirkstoff nachweisen. Andere Protein-Wirkstoffe werden in deutlich höheren Mengen formuliert; das HSA kann deshalb um so einfacher durch die erfindungsgemäße Zusammensetzung zur Stabilisierung ersetzt werden.

*Clostridium botulinum* Toxin Typ A (Handelsname Botox^{®}, Fa. Allergan; Dysport^{®}, Fa. Ipsen) wird seit vielen Jahren zur Therapie verschiedener Formen von Dystonien (z.B. Blepharospasmus, Torticollis), von Spastizitäten, zur Behandlung von Hyperhidrose, aber auch im kosmetischen Bereich zur Entfernung von Gesichtsfalten eingesetzt. Bei diesem Wirkstoff handelt es sich um einen Protein-Komplex, der von anaerob wachsenden Bakterien (*Clostridium botulinum*) synthetisiert wird. Das wirksame Agens in diesem Protein-Komplex ist ein Protein mit dem Molekulargewicht 150 kD, das Botulinum Neurotoxin (BoNT). Dieses Toxin bzw. Neurotoxin wirkt an der motorischen Endplatte und inhibiert die Übertragung des Nervenimpulses auf den Muskel und führt damit zu einer Paralyse dieses Muskels. Dieser Wirkungsmechanismus erlaubt es, das Neurotoxin bei Erkrankungen einzusetzen, bei denen die Reizübertragung pathologisch verändert ist, d. h. eine vermehrte Acetylcholin-Ausschüttung erfolgt.

Die auf dem Markt derzeit vertriebenen Botulinum-Toxine bzw. -Neurotoxine basieren alle auf dem Toxin-Komplex aus *Clostridium botulinum,* d. h. das Neurotoxin, das eigentlich wirksame Molekül, ist eingebettet in ein Ensemble aus Proteinen mit unterschiedlichen Molekulargewichten: dies sind verschiedene Hämagglutinine (15 kD, 19 kD, 35 kD, 52 kD) sowie ein nicht-toxisches nicht-hämagglutinierendes Protein (NTNH, 120 kD). Ohne diese sog. Schutzproteine ist das isolierte Neurotoxin sehr instabil und wird leicht durch Proteasen abgebaut. Die Schutzproteine bzw. komplexierenden Proteine sind also für die eigentliche Wirkung an der Nervenzelle überflüssig, spielen bei der Stabilisierung des empfindlichen Neurotoxins aber eine Rolle. Andererseits gibt es Hinweise, dass diese komplexierenden Proteine eine immunstimulierende Wirkung ausüben, die dafür verantwortlich sein könnte, dass 5-10 % der Patienten Antikörper entwickeln, die unweigerlich zum Ende der Therapie mit diesem Neurotoxin-Typ führen ("Secondary Nonresponder"). Ferner ist zu bedenken, dass Patienten mit Fremdprotein (den komplexierenden Proteinen) belastet werden, was pharmakologisch nicht zwingend erforderlich ist. Es ist daher sinnvoll, das reine, Komplexprotein-freie Neurotoxin als Wirkstoff einzusetzen, das aber wegen seiner geringen Dosis und Labilität einer besonders effizienten Formulierung bedarf.

Die Voraussetzung für die Verwendung des Komplexprotein-freien Neurotoxins (das Komplexprotein-freie Neurotoxin wird gelegentlich auch als hochreines Neurotoxin bezeichnet) als Wirkstoff in einem Arzneimittel bestand also darin, eine pharmazeutische Zusammensetzung zu entwickeln, die die Stabilität der biologischen Wirkung des hochreinen Neurotoxins über einen längeren Zeitraum gewährleistet. Diese Voraussetzung hat der Erfinder mit der Bereitstellung der erfindungsgemäßen Zusammensetzung zur Stabilisierung erfüllt.

Die beiden derzeit auf dem Markt befindlichen Arzneimittel auf Grundlage des Neurotoxins vom Typ A enthalten als essentiellen Stabilisator HSA (Botox^{®} enthält bei 100 Einheiten Wirkstoff 0.5 mg HSA und zusätzlich 0.9 mg Natriumchlorid, während Dysport^{®} bei 500 Einheiten Wirkstoff 0.125 mg HSA und außerdem 2.5 mg Lactose enthält). Das auf dem Toxin-Komplex Typ B basierende Neurobloc^{®} besteht mit 2000 Einheiten aus einer Lösung mit 500 µg/ml HSA, 0.01 M Natriumsuccinat und 0.1 M Natriumchlorid. Wie oben beschrieben dient das HSA insbesondere dazu, die Adsorption des Toxins an Gefäßwandungen (Glas-Fläschchen, Spritzen, Kanülen) zu verhindern und vor Denaturierung zu schützen. Ohne das Serumalbumin (oder ohne Substanzen, die dessen Effekt substituieren) geht das Toxin unweigerlich verloren. Das ist vor allem der Tatsache geschuldet, dass die Menge an Neurotoxin in diesen Arzneimitteln äußerst gering ist (Botox^{®} enthält 5 ng, Dysport^{®} 20 ng Toxin-Komplex pro Packungseinheit (Fläschchen)). Sofern kein anderes Protein anwesend ist, werden vom Toxin die genügend vorhandenen unspezifischen Proteinbindungsplätze eingenommen. Bei Anwesenheit von einem hohen Überschuss (> 50 000-fach) wie in den genannten Arzneimitteln werden vom HSA die Bindungsplätze belegt, so dass der Neurotoxin-Komplex in Lösung bleibt. Die Wahrscheinlichkeit, dass das hochreine Komplex-freie Neurotoxin an der festen Oberfläche des Behältnisses adsorbiert wird, ist erheblich größer, denn die Proteinmenge an reinem Neurotoxin für eine Dosis von 100 Einheiten beträgt lediglich 500 pg.

Patenanmeldung WO 01/58472 beschreibt eine Formulierung für den Komplex aus *Clostridium botulinum* vom Typ A, die im wesentlichen aus Hydroxyethylstärke besteht. Es werden Beispiele aufgeführt, in denen die Formulierung ein Jahr stabil ist. Mit dem hochreinen, also Komplexprotein-freien Neurotoxin werden keine Untersuchungen beschrieben. Es wird jedoch konstatiert, daß das Toxin nach Entfernen der Hämagglutinine eine signifikante Instabilität aufweist. Des weiteren wird zum hochreinen Botulinum-Toxin bemerkt, dass es so instabil sei, dass es für die Herstellung einer pharmazeutischen Zusammensetzung nur eine sehr eingeschränkte Brauchbarkeit aufweist. ("The toxin protein has a marked instablity upon removal of the hemagglutin protein" und "Pure botulinum toxin is so labile that it has limited practical utility to prepare a pharmaceutical composition.") Es wird jedoch nicht erwähnt, dass die beschriebene Formulierung auf der Basis von Hydroxyethylstärke auch zur Stabilisierung des hochreinen Neurotoxins effizient ist.

Eine HSA-haltige Formulierung von hochreinem Neurotoxin wird beschrieben in den US Patenten 5,512,547 und 5,756,468. Im ersten Patent wird eine Formulierung beschrieben, die HSA sowie Trehalose und Maltotriose oder verwandte Saccharide enthält. Das zweite Patent gibt eine Formulierung an, die in Ergänzung zu diesen Sacchariden noch Methionin oder Cystein enthält. Die Notwendigkeit, in einer Formulierung für das hochreine Neurotoxin HSA zu verwenden, ist gleichfalls in einer Publikation dargestellt (Goodenough at al (1992) Appl Environm Microbiol 58 3426-3428).

Die erfindungsgemäße pharmazeutische Zusammensetzung kann beispielsweise wie folgt hergestellt werden: Eine Lösung des Wirkstoffes (z.B. eines Neurotoxins aus *Clostridium botulinum*) wird mit der Zusammensetzung zur Stabilisierung (in Form einer wässrigen Lösung) verdünnt auf eine Konzentration von 1.0-1.2 ng/ml (≅ 200 Einheiten/ml) und anschließend sterilfiltriert. Jeweils 0.5 ml dieser Verdünnung werden in Fläschchen abgefüllt, lyophilisiert oder Vakuum-getrocknet und bis zum therapeutischen Einsatz gelagert. Ein Fläschchen enthält somit ein Lyophilisat oder Vakuum-getrocknetes Pulver mit etwa 100 Einheiten des Neurotoxins. Das Lyophilisat oder Pulver wird für die Applikation am Patienten je nach Indikation mit 2-8 ml WFI rekonstituiert. Die beschriebene Zusammensetzung zur Stabilisierung gemäß vorliegender Erfindung gewährleistet eine vollständige Wiederfindung des Protein-Wirkstoffes (des Neurotoxins) nach Verdünnen, Sterilfiltrieren, Abfüllen und Lyophilisieren. Das Lyophilisat ist bei 37°C mehr als 6 Monate stabil.

### Beispiel 1

Es sollte geprüft werden, ob die Verwendung der erfindungsgemäßen Zusammensetzung zur Stabilisierung im Vergleich zu einem Phosphatpuffer bzw. zu einer Zusammensetzung aus einem Phosphatpuffer und Polysorbat eine höhere Wiederfindung ermöglicht.

Alle verwendeten Hilfsstoffe (Excipients) wurden von Herstellern in pharmazeutischer Qualität bezogen. *Clostridium botulinum* Neurotoxin Typ A ist erhältlich bei List Biological Laboratories, Inc. Campell, California, USA bzw. wurde produziert gemäß DasGupta, B.R. (1984) Toxicon 3, 415-424.

Eine Lösung von *Clostridium botulinum* Neurotoxin Typ A (168 µg/ml) wurde mit einer Zusammensetzung zur Stabilisierung gemäß vorliegender Erfindung auf eine Konzentration von 0.5 ug/ml verdünnt. Die Zusammensetzung zur Stabilisierung war 50 mM in Bezug auf Asparaginsäure, Asparagin, Glutaminsäure und Glutamin, enthielt 0.05 Gew.-% Polysorbat 20 und wies einen pH von 7.5 auf.

Eine weitere Verdünnung auf 1.2 ng/ml (∼200 LD₅₀/ml) erfolgte mit verschiedenen Lösungen (siehe Tabelle 1). Nach einer Filtration über ein 0.22µ Filter wurden von diesen weiter verdünnten Lösungen jeweils 0.5 ml in ein Glas-Fläschchen (6R, Fa. Münnerstädt) abgefüllt und bei 37°C gelagert. Die Fläschchen wurden mit einen Gummistopfen verschlossen. Nach 15 h Stunden Lagerung wurde die Konzentration des Neurotoxins in den einzelnen Lösungen mittels eines herkömmlichen spezifischen Enzymimmunoassays (EIA) bestimmt.

**Tabelle 1**

| **Zusammensetzung** | **Konzentration bzw. Gehalt** | **Wiederfindung (%)** |
|---|---|---|
| **1. Lösung** | | |
| Na Phosphat | 50 mM | 0 |

| **2. Lösung** | | |
|---|---|---|
| Na Phosphat | 50 mM | 62.5 |
| Polysorbat 20 | 0.05 Gew.-% | |

| **3. Lösung** | | |
|---|---|---|
| Asparaginsäure | 50 mM | |
| Asparagin | 50 mM | 111 |
| Glutaminsäure | 50 mM | |
| Glutamin | 50 mM | |
| Polysorbat 20 | 0.05 Gew.-% | |
| EDTA | 0.5 mM | |

Die gewählte Formulierung ergab eine vollständige Wiederfindung nach einer Inkubation bei 37°C.

### Beispiel 2

Es sollte geprüft werden, ob eine erfindungsgemäße pharmazeutische Zusammensetzung mit 200 Einheiten Neurotoxin Typ A/ml (1,2 ng/ml) über einen längeren Zeitraum stabil ist.

Aus einer Stammlösung mit 0.5µg/ml *Clostridium botulinum* Neurotoxin Typ A wurde unter Verwendung der in Tabelle 2 angegebenen Zusammensetzungen, entsprechend Beispiel 1, eine Verdünnung mit einer Konzentration von 1.2 ng/ml hergestellt. Nach Sterilfiltration wurden je 0.5 ml dieser Zusammensetzungen in Fläschchen gefüllt, die mit einem Gummistopfen verschlossen wurden. Nach 15 h Lagerung bei 4°C und 37°C wurde die Menge an Neurotoxin Typ A im ELISA bestimmt.

Nach 8 Monaten Lagerung bei 4°C wurde die biologische Aktivität in den Fläschchen mittels eines ex vivo-Assays bestimmt. Dabei wurde die Aktivität der Zusammensetzungen mittels des Maus-Zwerchfell-Assays (Wohlfahrt K. et al. (1997) Naunyn-Schmiedebergs Arch. Pharmcol. 355, 225-340) bestimmt.

**Tabelle 2**

| **Zusammensetzung** | **Konzentration** | **15 h** | | **Wiederfindung (%) nach 8 Monaten (4°C)** |
|---|---|---|---|---|
| | **bzw. Gehalt** | **4°C** | **37°C** | |
| * Asparaginsäure | 50 mM | | | |
| Asparagin | 50 mM | | | |
| Glutaminsäure | 50 mM | 100 | 21 | 100 |
| Glutamin | 50 mM | | | |
| Polysorbat 80 | 0.05 Gew.-% | | | |
| Asparaginsäure | 50 mM | | | |
| Asparagin | 50 mM | 12 | 0 | - |
| Glutaminsäure | 50 mM | | | |
| Glutamin | 50 mM | | | |

| | | | | |
|---|---|---|---|---|
| *: nicht von den Ansprüchen umfasst | | | | |

Eine Lösung bestehend aus den Aminosäuren Asn, Asp, Gln und Glu (je 50 mM) und Polysorbat 80 war bei 4°C für mindestens 8 Monate stabil.

### Beispiel 3

Es sollte überprüft werden, welchen stabilisierenden Effekt unterschiedliche Gemische der Aminosäuren ausüben. Die pharmazeutischen Zusammensetzungen wurden wiederum auf eine Konzentration von 1.2 ng *Clostridium botulinum* Neurotoxin Typ A/ml (200 Einheiten/ml) eingestellt (Verdünnungen erfolgten nach Beispiel 1 bzw. mit den in Tab. 3 angegebenen Lösungen) und nach Sterilfiltration über einen 0.22µ Filter bei 4°C gelagert. Die Ergebnisse einer Neurotoxin-Bestimmung im Enzymimmunoassay sind in Tabelle 3 dargestellt.

**Tabelle 3**

| **Zusammensetzung** | **Konzentration bzw. Gehalt** | **pH-Wert** | **Wiederfindung (%)** |
|---|---|---|---|
| * Asparaginsäure | 50 mM | | |
| Asparagin | 50 mM | | |
| EDTA | 0.5 mM | 7.5 | 65 |
| Polysorbat 20 | 0.05 Gew.-% | | |
| Saccharose | 1 Gew.-% | | |
| * Glutaminsäure | 50 mM | | |
| Glutamin | 50 mM | | |
| EDTA | 0.5 mM | | |
| Polysorbat 20 | 0.05 Gew.-% | 7.5 | 12 |
| Saccharose | 1 Gew.-% | | |
| * Glutaminsäure | 50 mM | | |
| EDTA | 0.5 mM | | |
| Polysorbat 20 | 0.05 Gew.-% | 7.5 | 10 |
| Saccharose | 1 Gew.-% | | |
| Asparaginsäure | 50 mM | | |
| Asparagin | 50 mM | | |
| Glutaminsäure | 50 mM | | |
| Glutamin | 50 mM | 7.5 | 106 |
| EDTA | 0.5 mM | | |
| Polysorbat 20 | 0.05 Gew.-% | | |
| Saccharose | 1 Gew.-% | | |

| | | | |
|---|---|---|---|
| *: nicht von den Ansprüchen umfasst | | | |

Ein Gemisch aus allen vier Aminosäuren ergab eine vollständige Wiederfindung des Wirkstoffes.

### Beispiel 4

Es sollte überprüft werden, bei welchem pH-Wert die entwickelte Formulierung die höchste Wiederfindung liefert. Die Formulierung wurde bei pH-Werten von 6.0 bis 8.0 bei einer Konzentration von 1.2 mg/ml *Clostridium botulinum* Neurotoxin Typ A zubereitet und nach Filtration über einen 0.22 µ Filter bei 37°C gelagert. Die Ergebnisse der Neurotoxin-Bestimmung im Enzymimmunoassay zeigt Tabelle 4.

**Tabelle 4**

| **Zusammensetzung** | **Konzentration bzw. Gehalt** | **pH-Wert** | **Wiederfindung nach** | | |
|---|---|---|---|---|---|
| | | | **2 Tagen** | **9 Tagen** | **21 Tagen** |
| Asparaginsäure | 50 mM | | | | |
| Asparagin | 50 mM | | | | |
| Glutaminsäure | 50 mM | | | | |
| Glutamin | 50 mM | 6.0 | 100 | 95 | 78 |
| EDTA | 0.5 mM | | | | |
| Polysorbat 20 | 0.05 Gew.-% | | | | |
| Saccharose | 1 Gew.-% | | | | |
| Asparaginsäure | 50 mM | | | | |
| Asparagin | 50 mM | | | | |
| Glutaminsäure | 50 mM | | | | |
| Glutamin | 50 mM | 6.5 | 100 | 92 | 83 |
| EDTA | 0.5 mM | | | | |
| Polysorbat 20 | 0.05 Gew.-% | | | | |
| Saccharose | 1 Gew.-% | | | | |
| Asparaginsäure | 50 mM | | | | |
| Asparagin | 50 mM | | | | |
| Glutaminsäure | 50 mM | | | | |
| Glutamin | 50 mM | 7.0 | 100 | 85 | 75 |
| EDTA | 0.5 mM | | | | |
| Polysorbat 20 | 0.05 Gew.-% | | | | |
| Saccharose | 1 Gew.-% | | | | |
| Asparaginsäure | 50 mM | | | | |
| Asparagin | 50 mM | | | | |
| Glutaminsäure | 50 mM | | | | |
| Glutamin | 50 mM | 7.5 | 100 | 61 | 55 |
| EDTA | 0.5 mM | | | | |
| Polysorbat 20 | 0.05 Gew.-% | | | | |
| Saccharose | 1 Gew.-% | | | | |
| Asparaginsäure | 50 mM | | | | |
| Asparagin | 50 mM | | | | |
| Glutaminsäure | 50 mM | | | | |
| Glutamin | 50 mM | 8.0 | 85 | 17 | 6 |
| EDTA | 0.5 mM | | | | |
| Polysorbat 20 | 0.05 Gew.-% | | | | |
| Saccharose | 1 Gew.-% | | | | |

Bei pH-Werten von 6.0 und 6.5 ergaben sich die besten Wiederfindungen.

### Beispiel 5

Es sollte untersucht werden, bei welcher Konzentration der 4 Aminosäuren Asparaginsäure, Asparagin, Glutaminsäure und Glutamin die höchste Wiederfindung erzielt wird. Es wurde aus einer Verdünnung auf 0.5 µg/ml *Clostridium botulinum* Neurotoxin Typ A eine weitere Verdünnung auf 1.2 µg/ml hergestellt und nach Filtration über einen 0.22 µ Filter in GR Fläschchen in einer Dosierung von 0.5 ml pro Fläschchen abgefüllt. Nach Verschließung mit einem Gummistopfen wurde bei 4°C 15 h gelagert und anschließend die Menge an Neurotoxin/Fläschchen bestimmt.

**Tabelle 5 a**

| **Zusammensetzung** | **Konzentration bzw. Gehalt** | **pH-Wert** | **Wiederfindung (in %)** |
|---|---|---|---|
| Asparaginsäure | 200 mM | | |
| Asparagin | 200 mM | | |
| Glutaminsäure | 200 mM | | |
| Glutamin | 200 mM | 7.5 | 19 |
| Polysorbat 20 | 0.01 Gew.-% | | |
| EDTA | 0.5 mM | | |
| Saccharose | 5 Gew.-% | | |
| Asparaginsäure | 100 mM | | |
| Asparagin | 100 mM | | |
| Glutaminsäure | 100 mM | | |
| Glutamin | 100 mM | 7.5 | 56 |
| Polysorbat 20 | 0.01 Gew.-% | | |
| EDTA | 0.5 mM | | |
| Saccharose | 5 Gew.-% | | |
| Asparaginsäure | 50 mM | | |
| Asparagin | 50 mM | | |
| Glutaminsäure | 50 mM | | |
| Glutamin | 50 mM | 7.5 | 93 |
| Polysorbat 20 | 0.01 Gew.-% | | |
| EDTA | 0.5 mM | | |
| Saccharose | 5 Gew.-% | | |

**Tabelle 5 b**

| **Zusammensetzung** | **Konzentration bzw. Gehalt** | **pH-Wert** | **Wiederfindung (%)** |
|---|---|---|---|
| Asparaginsäure | 50 mM | | |
| Asparagin | 50 mM | | |
| Glutaminsäure | 50mM | | |
| Glutamin | 50 mM | 6.5 | 79 |
| EDTA | 0.5 mM | | |
| Polysorbat 20 | 0.2 Gew.-% | | |
| Saccharose | 5 Gew.-% | | |
| Asparaginsäure | 50 mM | | |
| Asparagin | 50 mM | | |
| Glutaminsäure | 50 mM | | |
| Glutamin | 50 mM | 6.5 | 86 |
| EDTA | 0,5 mM | | |
| Polysorbat 20 | 0.2 Gew.-% | | |
| Saccharose | 5 Gew.-% | | |
| Asparaginsäure | 20 mM | | |
| Asparagin | 20 mM | | |
| Glutaminsäure | 20 mM | | |
| Glutamin | 20 mM | 6.5 | 0 |
| EDTA | 0.5 mM | | |
| Polysorbat 20 | 6.2 Gew.-% | | |
| Saccharose | 5 Gew.-% | | |
| Asparaginsäure | 10 mM | | |
| Asparagin | 10 mM | | |
| Glutaminsäure | 10 mM | | |
| Glutamin | 10 mM | 6.5 | 0 |
| EDTA | 0.5 mM | | |
| Polysorbat 20 | 0.2 Gew.-% | | |
| Saccharose | 5 Gew.-% | | |

Für die flüssige Formulierung ergab sich eine Konzentration der Aminosäuren von 50 mM als effizient für die Wiederfindung des Wirkstoffes.

### Beispiel 6

Es wurde untersucht, welche Zusammensetzung an Aminosäuren die höchste Wiederfindung nach einer Gefriertrocknung ergibt, wobei in diesem Ansatz kein EDTA verwendet wurde. Die abgefüllte Lösung (0.5 ml) wurde gefriergetrocknet und über Nacht bei 4°C gelagert. Die Lyophilisate wurden in 0.5 ml Wasser für Injektionszwecke rekonstituiert. Die Neurotoxin-Konzentration wurde mittels Enzymimmunoassay bestimmt.

**Tabelle 6**

| **Zusammenfassung** | **Konzentration bzw. Gehalt** | **pH-Wert** | **Wiederfindung (%) im Lyophilisat** |
|---|---|---|---|
| * Asparaginsäure | 100 mM | | |
| Asparagin | 100 mM | 6.5 | 0 |
| Saccharose | 5 Gew.-% | | |
| Polysorbat 80 | 0.2 Gew.-% | | |
| * Asparaginsäure | 50 mM | | |
| Asparagin | 50 mM | 6.5 | 20 |
| Saccharose | 5 Gew.-% | | |
| Polysorbat 80 | 0.2 Gew.-% | | |
| Asparaginsäure | 100 mM | | |
| Glutaminsäure | 100 mM | | |
| Asparagin | 100 mM | 6.5 | 9.3 |
| Saccharose | 5 Gew.-% | | |
| Polysorbat 80 | 0.2 Gew.-% | | |
| Asparaginsäure | 50 mM | | |
| Glutaminsäure | 50 mM | | |
| Asparagin | 50 mM | 6.5 | 56 |
| Saccharose | 5 Gew.-% | | |
| Polysorbat 80 | 0.2 Gew.-% | | |
| * Asparaginsäure | 100 mM | | |
| Glutaminsäure | 100 mM | 6.5 | 20 |
| Saccharose | 5 Gew.-% | | |
| Polysorbat 80 | 0.2 Gew.-% | | |
| Asparaginsäure | 50 mM | | |
| Asparagin | 50 mM | | |
| Glutaminsäure | 50 mM | 6.5 | 87 |
| Glutamin | 50 mM | | |
| Saccharose | 5 Gew.-% | | |
| Polysorbat 80 | 0.2 Gew.-% | | |

| | | | |
|---|---|---|---|
| *: nicht von den Ansprüchen umfasst | | | |

Die höchste Wiederfindung des Wirkstoffes in Lyophilisaten ergab sich, wenn alle 4 Aminosäuren in der Zusammensetzung vorlagen, und dies in einer Konzentration von 50 mM.

### Beispiel 7

Aus einer Vorverdünnung des *Clostridium botulinum* Neurotoxins Typ A in einer Lösung, die je 50 mM an Asparaginsäure, Asparagin, Glutaminsäure und Glutamin, und 0.5 mM an EDTA war, und die außerdem 0.2 Gew.-% Polysorbat 80 und 5 Gew.-% Saccharose sowie einen pH von 6.5 aufwies, wurde eine Endverdünnung von 1.26 ng Neurotoxin Typ A/ml (200 U/ml) in einer Lösung mit gleicher Zusammensetzung hergestellt und über 0.22 µ Membranfilter filtriert. Davon wurden je 0.5 ml in 6R Glasfläschchen pipettiert und anschließend lyophilisiert. Das Lyophilisat wurde in WFI gelöst. Der Gehalt an Wirkstoff (Neurotoxin Typ A) wurde im Enzymimmunoassy bestimmt. Es wurden 96 Gew.-% des Wirkstoffes nachgewiesen. Zur Überprüfung der biologischen Wirksamkeit des wiedergefundenen Wirkstoffes wurde das Lyophilisat gelöst und am Zwerchfell getestet. Das Lyophilisat eines Fläschchens enthielt 110 Einheiten (entsprechend 110 % Wiederfindung).

### Beispiel 8

Analog Beispiel 7 wurden Lyophilisate hergestellt und anschließend bei 37°C gelagert. Nach 3 Monaten wurde der Gehalt an Wirkstoff im Immunoassay bestimmt. Es wurden 94 Gew.-% des eingesetzten Wirkstoffs nachgewiesen. Die Überprüfung der Aktivität/Fläschchen im biologischen Assay (Zwerchfell-Assay) ergab einen Gehalt von 102 Einheiten/Fläschchen.

### Beispiel 9 (nicht von den Ansprüchen umfasst)

Aus einer Vorverdünnung von Interferon beta in einer Lösung (die für einen Ansatz ohne, und für einen anderen Ansatz 0.5 mM an EDTA war), die je 50 mM an Asparaginsäure, Asparagin, Glutaminsäure und Glutamin war, und die außerdem 0.2 Gew.-% Polysorbat 80 und 5 Gew.-% Saccharose sowie einen pH von 7.0 aufwies, wurden Endverdünnungen mit 20 µg/ml (4 Mio internationale Einheiten/ml) in Lösungen mit entsprechender Zusammensetzung hergestellt und über einen 0.22 µ Membranfilter filtriert. Von den Filtraten wurden je 1 ml in 6R-Glasfläschchen pipettiert und anschließend lyophilisiert. Die Lyophilisate wurden in WFI gelöst. Der Gehalt an Wirkstoff (Interferon beta) wurde im Enzymimmunoassay bestimmt. Es wurden 18.8 (ohne EDTA) bzw. 19.6 µg (0.5 mM EDTA) des Wirkstoffes nachgewiesen. Zur Überprüfung der biologischen Wirksamkeit des wiedergefundenen Wirkstoffes wurden die Lyophilisat gelöst und die Aktivität im herkömmlichen Bioassay bestimmt (Hemmung des cytopathischen Effekts an VERO-Zellen im Vergleich zum Referenzstandard). Es wurden 94 bzw. 95% der eingesetzten biologischen Aktivität wiedergefunden.

### Beispiel 10

Aus einer Vorverdünnung von Blutgerinnungsfaktor VIII in einer Lösung, die je 50 mM an Asparaginsäure, Asparagin, Glutaminsäure und Glutamin und 0.5 mM an EDTA war, und die außerdem 0.2 Gew.-% Polysorbat und 5 Gew.-% Saccharose sowie einen pH von 7.3 aufwies, wurde eine Endverdünnung mit 250 internationale Einheiten/ml in einer Lösung mit gleicher Zusammensetzung hergestellt und über einen 0.22 µ Membranfilter filtriert. In einem Ansatz wurde Polysorbat 20, im anderen Polysorbat 80 verwendet. Von den erhaltenen Filtraten wurden je 1 ml in 6R-Glasfläschchen pipettiert und anschließend lyophilisiert. Die Lyophilisate wurden in WFI gelöst. Der Gehalt an Wirkstoff (Blutgerinnnungsfaktor VIII) wurde im herkömmlichen Gerinnungstest bestimmt. Es wurden 238 (P 20) bzw. 245 (P 80) internationale Einheiten pro Fläschchen nachgewiesen.

### Beispiel 11

Aus einer Vorverdünnung von Streptokinase in Lösung, die je 50 mM an Asparaginsäure, Asparagin, Glutaminsäure und Glutamin und 0.5 mM an EDTA war, und die außerdem 0.2 Gew.-% Polysorbat 80 und 2.5, 5 bzw. 7.5 Gew.-% Saccharose sowie einen pH von 7.0 aufwiesen, wurden eine Endverdünnung auf 250,000 internationale Einheiten/ml in Lösungen mit entsprechender Zusammensetzung hergestellt und über einen 0.22 µ Membranfilter filtriert. Von den Filtraten wurden je 1 ml in 6R-Glasfläschchen pipettiert und anschließend lyophilisiert. Die Lyophilisate wurden in WFI gelöst. Der Gehalt an Wirkstoff (Streptokinase) wurde im Standard-Fibrinolyse-Test bestimmt. Es wurden 236,500, 247,000 bzw. 242,500 internationale Einheiten pro Fläschchen nachgewiesen.

## Patentansprüche

1. Pharmazeutische Zusammensetzung, umfassend einen Protein-Wirkstoff und eine Zusammensetzung zur Stabilisierung wobei die Zusammensetzung zur Stabilisierung eine Zusammensetzung zur Stabilisierung von Protein-Wirkstoffen in Arzneimitteln ist, die frei ist von humanem Serumalbumin, umfassend die folgenden beiden Komponenten:
a) eine oberflächenaktive Substanz, vorzugsweise ein nicht-ionisches Detergens (Tensid), und
b) ein Gemisch von mindestens drei Aminosäuren, wobei das Gemisch aus
(i) Asparaginsäure, Asparagin und Glutaminsäure,
(ii) Asparaginsäure, Asparagin und Glutamin,
(iii) Asparaginsäure, Glutaminsäure und Glutamin,
(iv) Asparagin, Glutaminsäure und Glutamin oder
(v) Asparaginsäure, Asparagin, Glutaminsäure und Glutamin
besteht
und wobei den Protein-wirkstoff ein Gerinnungsfaktor wie der Faktor VIII (das antihämophile Globulin), ein Enzym wie eine Urokinase oder Streptokinase, ein Plasminogenaktivator oder ein hochreines Neurotoxin bzw. ein Neurotoxin-Komplex aus *Clostridium botulinum,* insbesondere aus *Clostridium botulinum* der Typen A oder B ist.

2. Die pharmazeutische Zusammensetzung nach Anspruch 1, wobei die Zusammensetzung zur Stabilisierung weiterhin wenigstens eine der folgenden Komponente umfasst:
c) ein Disaccharid, vorzugsweise Sucrose (Saccharose, Rohrzucker), Trehalose oder Lactose,
d) Ethylendiamintetraessigsäure (EDTA), vorzugsweise in Form eines ihrer Salze wie des Na₄-EDTA.

3. Die pharmazeutische Zusammensetzung nach Anspruch 1 oder 2, wobei die Zusammensetzung zur Stabilisierung die Komponenten a), b) und c), die Komponenten a), b) und d) oder die Komponenten a), b), c) und d) umfasst.

4. Die pharmazeutische Zusammensetzung nach einem der vorhergehenden Ansprüche, wobei die Zusammensetzung zur Stabilisierung entweder m wässrigen Medien löslich ist oder als wässrige Lösung vorliegt.

5. Die pharmazeutische Zusammensetzung nach einem der Ansprüche 1-4, wobei die pharmazeutische Zusammensetzung als gefrier- oder Vakuum-getrocknetes Pulver vorliegt, das in wässrigen Medien löslich ist.

6. Die Zusammensetzung nach einem der Ansprüche 1 bis 5, wobei die Konzentrationen der einzelnen Aminosäuren jeweils 20 bis 200 mM, besser 20 bis 100 mM, insbesondere 50 mM, sind.

7. Die Zusammensetzung nach einem der Ansprüche 1 bis 6, wobei die oberflächenaktive Substanz ein nicht-ionisches Detergens ist.

8. Die Zusammensetzung nach Anspruch 7, wobei das nicht-ionische Detergens ein Polysörbat wie Polysorbat 20 oder Polysörbat 80 oder ein Poloxamer wie Poloxamer 184 oder 188 ist.

9. Die Zusammensetzung nach einem der Ansprüche 6 bis 8, wobei das Disaccharid Saccharose, Trehalose oder Lactose ist.

10. Die Zusammensetzung nach einem der Ansprüche 6 bis 9, wobei der pH-Wert der Zusammensetzung in Lösung bei 5.0 bis 8.5, insbesondere bei 6.0 bis 8.0, speziell bei 6.0 bis 7.0 bzw. 6.5 liegt.

## Claims

1. Pharmaceutical composition comprising a protein agent and a composition for stabilization, wherein the composition for stabilization is a composition free of human serum albumin for stabilization of protein agents in pharmaceuticals, the composition comprising the following two constituents:
a) a surface active substance, preferably a non-ionic detergent (tenside), and
b) a mixture of at least three amino acids, wherein the mixture consists of
(i) aspartic acid, asparagine and glutamic acid,
(ii) aspartic acid, asparagine and glutamine,
(iii) aspartic acid, glutamic acid and glutamine,
(iv) asparagine, glutamic acid and glutamine or
(v) aspartic acid, asparagine, glutamic acid and glutamine, and
wherein the protein agent is a coagulation factor such as factor VIII (the antihemophilic globulin), an enzyme such as urokinase or streptokinase, a plasminogen activator, or an ultra pure neurotoxin or a neurotoxin complex, respectively, from *Clostridium botulinum,* in particular from *Clostridium botulinum* type A or B..

2. The pharmaceutical composition of claim 1, wherein the composition for stabilization further comprises at least one of the following constituents:
c) a disaccharide, preferably sucrose (cane sugar), trehalose or lactose,
d) ethylenediaminetetraacetic acid (EDTA), preferably in form of one of its salts such as Na₄-EDTA.

3. The pharmaceutical composition according to claim 1 or 2, wherein the composition for stabilization comprises the constituents a), b) and c), the constituents a), b) and d) or the constituents a), b), c) and d).

4. The pharmaceutical composition according to any one of the preceding claims, wherein the composition for stabilization is either soluble in aqueous media or is present as aqueous solution.

5. The pharmaceutical composition according to any one of claims 1 - 4, wherein the pharmaceutical composition is present as a freeze-dried or vacuum-dried powder, which is soluble in aqueous media.

6. The composition according to any one of claims 1 to 5, wherein the concentrations of the individual amino acids are in each case 20 to 200 mM, more preferred 20 to 100 mM, in particular 50 mM.

7. The composition according to any one of claims 1 to 6, wherein the surface active substance is a non-ionic detergent.

8. The composition according to claim 7, wherein the non-ionic detergent is a polysorbate such as polysorbate 20 or polysorbate 80 or a poloxamer such as poloxamer 184 or 188.

9. The composition according to any one of claims 6 to 8, wherein the disaccharide is sucrose, trehalose or lactose.

10. The composition according to any one of claims 6 to 9, wherein the pH value of the composition in solution is 5.0 to 8.5, in particular 6.0 to 8.0, preferably 6.0 to 7.0 or 6.5, respectively.

## Revendications

1. Composition pharmaceutique comprenant un principe actif de protéine et une composition pour la stabilisation, dans laquelle la composition pour la stabilisation est une composition pour la stabilisation de principes actifs de protéine dans des médicaments, qui est exempte d'albumine sérique humaine, comprenant les deux composants suivants :
a) une substance tensioactive, de préférence un détergent non ionique (tensioactif), et
b) un mélange d'au moins trois acides aminés, dans lequel le mélange se compose
(i) d'acide asparaginique, d'asparagine et d'acide glutamique,
(ii) d'acide asparaginique, d'asparagine et de glutamine,
(iii) d'acide asparaginique, d'acide glutamique, et de glutamine,
(iv) d'asparagine, d'acide glutamique et de glutamine ou
(v) d'acide asparaginique, d'asparagine, d'acide glutamique, et de glutamine,
et dans laquelle le principe actif de protéine est un facteur de coagulation comme le facteur VIII (la globuline anti-hémophile), une enzyme comme une urokinase ou streptokinase, un activateur du plasminogène ou une neurotoxine hautement pure ou un complexe de neurotoxine constitué de *Clostridium botulinum,* en particulier *Clostridium botulinum* des types A ou B.

2. Composition pharmaceutique selon la revendication 1, dans laquelle la composition pour la stabilisation comprend en outre au moins un des composants suivants :
c) un disaccharide, de préférence du saccharose (saccharose, sucre de canne), du tréhalose ou du lactose,
d) de l'acide éthylènediamine tétraacétique (EDTA), de préférence sous la forme d'un de ses sels comme le Na₄-EDTA.

3. Composition pharmaceutique selon la revendication 1 ou 2, dans laquelle la composition pour la stabilisation comprend les composants a), b) et c), les composants a), b) et d) ou les composants a), b), c) et d).

4. Composition pharmaceutique selon l'une quelconque des revendications précédentes, dans laquelle la composition pour la stabilisation est soit soluble en milieux aqueux soit se présente sous forme de solution aqueuse.

5. Composition pharmaceutique selon l'une des revendications 1 à 4, dans laquelle la composition pharmaceutique se présente sous la forme d'une poudre lyophilisée ou séchée sous vide, qui est soluble en milieux aqueux.

6. Composition pharmaceutique selon l'une des revendications 1 à 5, dans laquelle les concentrations des différents acides aminés sont respectivement de 20 à 200 mM, préférentiellement de 20 à 100 mM, en particulier de 50 mM.

7. Composition pharmaceutique selon l'une des revendications 1 à 6, dans laquelle la substance tensioactive est un détergent non ionique.

8. Composition pharmaceutique selon la revendication 7, dans laquelle le détergent non ionique est un polysorbate comme le polysorbate 20 ou le polysorbate 80 ou un poloxamère comme le poloxamère 184 ou 188.

9. Composition pharmaceutique selon l'une des revendications 6 à 8, dans laquelle le disaccharide est le saccharose, le tréhalose ou le lactose.

10. Composition pharmaceutique selon l'une des revendications 6 à 9, dans laquelle le pH de la composition en solution se situe aux environs de 5,0 à 8,5, en particulier aux environs de 6,0 à 8,0, spécifiquement aux environs de 6,0 à 7,0 ou 6,5.
